# EUROPEAN PATENT APPLICATION

(11) **EP 4 265 739 A1**
(43) Date of publication of application: **25.10.2023**
(21) Application number: 22762537.3
(22) Date of filing: 02.03.2022
(51) Int. Cl.: C12Q 1/6886

(54) **GENE COMBINATION FOR HUMAN TUMOR GRADING, AND USE THEREOF**

(30) Priority: 02.03.2021 CN 202110232095; 26.03.2021 CN 202110332750; 06.07.2021 CN 202110762353
(71) Applicant: Peking University First Hospital, Beijing 100034 (CN)
(72) Inventor: XIONG, Gengyan, Beijing 100034 (CN); HE, Shiming, Beijing 100034 (CN); HE, Zhisong, Beijing 100034 (CN); ZHOU, Liqun, Beijing 100034 (CN)
(74) Representative: J A Kemp LLP
(86) International application number: PCT/CN2022/078709
(87) International publication number: WO 2022/184073

(57) **Abstract**

The present invention relates to the field of tumor grade detection. Specifically disclosed are a gene combination for human tumor grading, and a use thereof. The gene combination for human tumor grading consists of a gene set A and a gene fragment set B. The gene combination for human tumor grading is obtained from high-throughput sequencing data of an actual renal cancer case of the Peking University First Hospital by means of specific pairing and clustering, is derived from real data, and can be used for malignancy degree grading and prognosis prediction for renal cancer and pan-cancer.

## Description

### CROSS REFERENCE TO RELATED APPLICATIONS

The present application claims priority to Chinese Patent Application No. 202110232095X, entitled "gene combination for human tumor grading, and a use thereof", and filed to the China National Intellectual Property Administration on March 2, 2021, the entire contents of which are incorporated herein by reference; the present application also claims priority to Chinese Patent Application No. 2021103327509, entitled "gene combination for human tumor grading, and a use thereof", and filed to the China National Intellectual Property Administration on March 26, 2021, the entire contents of which are incorporated herein by reference; the present application also claims priority to Chinese Patent Application No. 2021107623535, entitled "gene combination for human tumor grading, and a use thereof", and filed to the China National Intellectual Property Administration on July 6, 2021, the entire contents of which are incorporated herein by reference.

### TECHNICAL FIELD

The present invention relates to the field of tumor grade detection, in particular to a gene combination for human tumor grading, and a use thereof.

### BACKGROUND

Renal cancer is a common malignant tumor of urinary system. In recent years, the incidence of renal cancer has increased year by year, accounting for 2% - 3% of adult malignant tumors, and increasing at a rate of about 2.5% per year. Renal cancer lacks typical clinical features in the early stage. Renal cancer with clinical symptoms is often in advanced stage. The treatment of renal cancer is a comprehensive treatment based on surgery. In the treatment, it is often necessary to grade the malignant degree of renal cancer tissue to help doctors judge the progress and prognosis of the disease, and formulate further treatment plans. Fuhrman nuclear grading is the most widely used grading system for malignant degree of renal cancer at present.

The Fuhrman nuclear grading system was proposed in 1982. The malignant degree and risk of the tumor were graded and evaluated through the Fuhrman grading system. According to the Fuhrman grading standard, renal cancer is divided into four levels: G1 (well-differentiated), G2 (moderately well-differentiated), G3 (moderately differentiated), and G4 (poorly differentiated or undifferentiated). With the increase of the level, the malignant degree of renal cancer is higher, and the risk of recurrence and metastasis after treatment is also increased. However, the Fuhrman grading system is a pure pathological image classification system, which has the following defects: 1. It needs to be judged according to the personal experience of the pathologist, which has a certain subjectivity, and there are huge differences between different pathologists; 2. G2 pathological images and G3 pathological images have a small degree of discrimination, and it is difficult to classify the two. The above defects are likely to lead to inaccurate grading of the malignant degree of the disease, errors in the judgment of disease progress and prognosis, and affect the diagnosis and treatment of the disease.

With the maturity and promotion of the next-generation sequencing technology, the method of using gene detection for diagnosing diseases has attracted widespread attention. For example, through whole exome sequencing, the mutation and copy number variation of marker genes are detected for diagnosing tumors or judging tumor progression. This method overcomes the shortcomings of subjectivity and difficulty in traditional tumor grading, and is of great significance for early diagnosis, treatment and prognosis of tumors. However, the existing gene combinations and methods based on the next-generation sequencing technology and used for grading the malignancy of renal cancer lack external validation, the malignancy grading is unreliable, and cannot be applied clinically. Therefore, it is urgent to find a new tumor malignancy and risk grading system based on specific gene detection.

### SUMMARY OF THE INVENTION

Therefore, the technical problem to be solved by present invention is to provide a gene combination for grading renal cancer and use thereof, which can grade the malignancy of renal cancer and can be used for prognostic prediction of renal cancer patients. In order to achieve this object, the present invention uses whole exome sequencing technology to screen the specially screened and grouped renal cancer patient data of the Peking University First Hospital, and finally obtain the gene combination, which is used for grading the malignant degree of renal cancer and predicting the prognosis, providing clinicians and patients with more accurate information on the malignant degree of renal cancer and disease prediction.

According to one aspect of the present invention, the present invention provides a gene combination for human tumor grading, the gene combination consists of a gene set A and a gene fragment set B; the gene set A comprises at least one selected from ASAH1, ASXL1, BCOR, BRAF, CALML6, CCDC136, CIDEC, COX18, CSF1R, CYP3A5, DEK, DNMT3A, EGR1, FAM71E2, FGFR1, FKBP7, FLT1, FLT3, FLT4, GLIS1, IDH2, IFITM3, IMMT, KDR, KIT, KMT2A, KNOP1, KRT76, KRT9, KRTAP10-10, KRTAP10-8, MAF, MECOM, MFRP, MLLT3, MNS1, MRTFA, MTOR, MYH11, NF1, NUP214, PDGFRA, PDGFRB, PML, PRB2, PROSER3, RAF1, RARA, RBM15, RET, REXO1, RPN1, RUNX1T1, SCYL1, SLC16A6, SRC, STAG2, TCEAL5, TET2, TMEM82, TP53, TRIM26, U2AF1, U2AF2, UGT1A1, USP35, VEGFA, WBP2NL, WDR44, ZNF20, ZNF700 and ZRSR2; the gene fragment set B comprises at least one selected from chr2:179479501-179610249, chr2:207989501-208000249, chr2:219719501-219840249, chr2:3679501-3700249, chr3:126249501-126270249, chr3:129319501-129330249, chr3:138659501-138770249, chr3:183999501-184020249, chr4:1189501-1230249, chr4:8579501-8590249, chr4:9319501-9330249, chr5:150899501-150940249, chr6:147819501-147840249, chr6:157089501-157110249, chr6:164889501-164900249, chr6:20399501-20410249, chr6:26519501-26530249, chr6:71659501-71670249, chr6:73329501-73340249, chr7:100539501-100560249, chr8:1939501-1960249, chr8:21999501-22070249, chr8:29189501-29200249, chr9:91789501-91800249, chr10:99419501-99440249, chr11:17739501-17760249, chr11:63329501-63350249, chr12:169501-250249, chr12:54329501-54350249, chr12:63179501-63550249, chr12:7269501-7310249, chr13:114519501-114530249, chr15:73649501-73670249, chr15:74209501-74220249, chr15:78409501-78430249, chr15:83859501-83880249, chr18:8809501-8820249, chr19:24059501-24070249, chr19:4229501-4250249, chr19:46879501-46900249, chr20:22559501-22570249, chr20:62189501-62200249, chr21:45949501-46110249, chr22:19499501-19760249, chr22:36649501-38700249 and chr22:46309501-47080249; and the position of the gene fragment in the gene fragment set B is annotated with GRCh37 as the standard. In GRCh38 or a new version of the human reference genome that appears in the future, its numbers may change, but the objective fragment positions pointed to and the genes that can be used for detection will not change.

Optionally, the detailed genes included in the gene fragment set B are as follows:

**Table 1 Gene Fragment Set B**

| position of gene fragment | genes available for detection |
|---|---|
| chr2:179479501-179610249 | at least one of TTN, MIR548N and LOC100506866 |
| chr2:207989501-208000249 | KLF7 |
| chr2:219719501-219840249 | at least one of WNT6, CDK5R2 and WNT10A |
| chr2:3679501-3700249 | COLEC11 |
| chr3:126249501-126270249 | at least one of C3orf22 and CHST13 |
| chr3:129319501-129330249 | PLXND 1 |
| chr3:138659501-138770249 | at least one of FOXL2, PRR23B, PRR23C, C3orf72 and PRR23A |
| chr3:183999501-184020249 | at least one of PSMD2 and ECE2 |
| chr4:1189501-1230249 | at least one of CTBP1, SPON2 and LOC100130872 |
| chr4:8579501-8590249 | GPR78 |
| chr4:9319501-9330249 | at least one of LOC728369, LOC728373, LOC728379, USP17L5, LOC728393, LOC728400 and LOC728405 |
| chr5:150899501-150940249 | FAT2 |
| chr6:147819501-147840249 | SAMD5 |
| chr6:157089501-157110249 | at least one of ARID1B and MIR4466 |
| chr6:164889501-164900249 | C6orf118 |
| chr6:20399501-20410249 | E2F3 |
| chr6:26519501-26530249 | HCG11 |
| chr6:71659501-71670249 | B3GAT2 |
| chr6:73329501-73340249 | KCNQ5 |
| chr7:100539501-100560249 | ACHE |
| chr8:1939501-1960249 | KBTBD 11 |
| chr8:21999501-22070249 | at least one of BMP1, SFTPC and LGI3 |
| chr8:29189501-29200249 | DUSP4 |
| chr9:91789501-91800249 | SHC3 |
| chr 10:99419501-99440249 | at least one of PI4K2A and AVPI1 |
| chr11:17739501-17760249 | at least one of KCNC1 and MYOD1 |
| chr11:63329501-63350249 | at least one of PLA2G16 and PLAAT2 |
| chr12:169501-250249 | at least one of IQSEC3 and LOC574538 |
| chr12:54329501-54350249 | at least one of HOXC12 and HOXC13 |
| chr12:63179501-63550249 | at least one of AVPR1A and PPM1H |
| chr12:7269501-7310249 | at least one of CLSTN3, RBP5 and MATL2963 |
| chr13:114519501-114530249 | GAS6 |
| chr15:73649501-73670249 | HCN4 |
| chr15:74209501-74220249 | at least one of LOXL1 and LOC100287616 |
| chr15:78409501-78430249 | CIB2 |
| chr15:83859501-83880249 | HDGFL3 |
| chr18:8809501-8820249 | MTCL1 |
| chr 19:24059501-24070249 | ZNF726 |
| chr19:4229501-4250249 | at least one of EBI3, CCDC94 |
| chr19:46879501-46900249 | PPP5C |
| chr20:22559501-22570249 | FOXA2 |
| chr20:62189501-62200249 | HELZ2 |
| chr21:45949501-46110249 | at least one of TSPEAR, KRTAP12-2, KRTAP12-1, KRTAP10-10, KRTAP10-4, KRTAP10-6, KRTAP10-7, KRTAP10-9, KRTAP10-1, KRTAP10-11, KRTAP10-2, KRTAP10-5, KRTAP10-8, KRTAP10-3, KRTAP12-3 and KRTAP12-4 |
| chr22:19499501-19760249 | at least one of GP1BB, SEPTIN5, TBX1, CLDN5, CDC45, LOC150185 and SEPT5-GP1BB |
| chr22:36649501-38700249 | at least one of hsa-mir-659, CSF2RB, CSNK1E, H1F0, IL2RB, LGALS1, LGALS2, MFNG, MPST, MYH9, NCF4, POLR2F, PVALB, RAC2, SOX10, SSTR3, TST, PLA2G6, GALR3, APOL1, EIF3D, PICK1, CACNG2, IFT27, TRIOBP, CDC42EP1, GCAT, SLC16A8, SH3BP1, MAFF, TXN2, TMEM184B, GGA1, CYTH4, CARDIO, EIF3L, PDXP, NOL12, KCTD17, FOXRED2, BAIAP2L2, C22orf23, MICALL1, ELFN2, C1QTNF6, ANKRD54, TMPRSS6, C22orf33, MIR658, MIR659, LOC100506241 and MIR4534 |
| chr22:46309501-47080249 | at least one of hsa-let-7b, PPARA, WNT7B, CELSR1, PKDREJ, GRAMD4, GTSE1, TTC38, C22orf26, TRMU, LOC150381, C22orf40, CN5H6.4, MIRLET7BHG, MIRLET7A3, MIRLET7B, LOC730668, LOC100271722, MIR3619 and MIR4763 |

Optionally, the gene set A comprises at least one selected from ASAH1, CCDC136, FAM71E2, IFITM3, KRT9, PRB2, PROSER3, TCEAL5, U2AF2, USP35, WDR44 and ZNF700.

Optionally, the gene fragment set B comprises at least one selected from chr2:179479501-179610249, chr2:207989501-208000249, chr2:21971-19840249, chr3:126249501-126270249, chr3:129319501-129330249, chr3:138659501-138770249, chr3:183999501-184020249, chr5:150899501-150940249, chr7: 100539501-100560249 and chr13: 114519501-114530249.

According to another aspect of the present invention, the present invention provides use of the gene combination above in the manufacture of products for human tumor grade detection.

Optionally, the tumor is a tumor of the urinary system or pan-cancer.

Optionally, the tumor of the urinary system is malignant tumor of the urinary system.

Optionally, the tumor of the urinary system is renal cancer.

Optionally, the pan-cancer is a cancer type in TCGA pan-cancer data.

Optionally, the tumor grading refers to the judgment of tumor malignancy and the prediction of tumor prognosis.

Optionally, the tumor grading is divided into a high-risk group and a low-risk group.

Optionally, the product comprises primers, probes, reagents, kits, gene chips or detection systems for detecting the genotypes of the genes in the gene combination.

Optionally, the product is used for detecting exon and related intron regions of genes in gene set A and gene fragment set B.

According to another aspect of the present invention, the present invention provides the method for grading the above tumors comprises the following steps:
Step S1: evaluating the gene mutation and gene copy number variation of genes contained in the gene set A in the cancer cell tissue, and evaluating the gene copy number variation of the gene fragment set B in the cancer cell tissue; and
Step S2: judging the degree of malignancy of the cancer and predicting the prognosis of the tumor, based on the evaluation results of step S1.

Optionally, the gene mutation comprises base substitution mutation, deletion mutation, insertion mutation and/or fusion mutation, and the gene copy number variation comprises gene copy number increase and/or gene copy number decrease.

Optionally, in the step S1, by comparing the sequencing data of the tumor tissue and the normal tissue, it is used for evaluating the gene mutation and copy number variation of the genes contained in the gene set A, while evaluating the copy number variation of the gene fragment set B.

Optionally, in step S2, if at least one gene in gene set A has gene mutation or copy number variation, or at least one fragment in gene fragment set B has copy number increase, the tumor is graded as the high-risk group; on the contrary, there is no gene mutation or copy number variation in gene set A, and no gene copy number increase in any fragment in gene fragment set B, and the tumor is graded as the low-risk group.

Optionally, any gene fragments are selected from the gene combination for combination to form a new gene combination, and the same tumor grading method is used for grading tumor malignancy and predicting tumor prognosis, so as to guide clinical diagnosis and treatment.

The technical solution of present invention has the following advantages:
1. The detection gene combination of present invention is obtained from the high-throughput sequencing data of the actual renal cancer cases in the Peking University First Hospital through specific paired clustering analysis. The real data has higher reliability and credibility, and can accurately grade the malignancy and predict the prognosis of renal cancer and pan-cancer.
2. The gene combination of present invention comprises the diversity of the gene combination, from which multiple gene combinations can be selected for judging the malignant degree of renal cancer and pan-cancer, and for different clinical situations.
3. Compared with whole exome sequencing, the present invention performs targeted sequencing analysis on specific genes and DNA fragments, which can significantly improve the sequencing depth and accuracy at the same cost, and can significantly save costs and have wide applicability at the same sequencing depth and accuracy.
4. Compared with the traditional Fuhrman pathological grading system, the present invention is completely unaffected by the subjective impression of pathologists, and has excellent objectivity and reliability.

### BRIEF DESCRIPTION OF THE DRAWINGS

In order to illustrate the technical solutions in the specific embodiments of the present invention or in the prior art more clearly, the drawings to be used in the description of the specific embodiments or the prior art will be briefly introduced below. Obviously, the drawings in the following description show some of the embodiments of the present invention, and those skilled in the art may also obtain other drawings from these drawings without creative efforts.
Figure 1 is the Kaplan-Meier survival analysis diagram with tumor specific survival as the primary endpoint after the gene combination in Example 1 of the present invention is used for grading the malignant degree of renal cancer in Experiment Example 1 of the present invention;
Figure 2 is the Kaplan-Meier survival analysis diagram with progression-free survival as the primary endpoint after the gene combination in Example 1 of the present invention is used for grading the malignant degree of renal cancer in Experiment Example 1 of the present invention;
Figure 3 is the Kaplan-Meier survival analysis diagram with overall survival as the primary endpoint after the gene combination in Example 1 of the present invention is used for grading the malignant degree of renal cancer in Experiment Example 1 of the present invention;
Figure 4 is the Kaplan-Meier survival analysis diagram with tumor specific survival as the primary endpoint after the gene combination 1 of the present invention is used for grading the malignant degree of renal cancer in Experiment Example 2 of the present invention;
Figure 5 is the Kaplan-Meier survival analysis diagram with progression-free survival as the primary endpoint after the gene combination 1 of the present invention is used for grading the malignant degree of renal cancer in Experiment Example 2 of the present invention;
Figure 6 is the Kaplan-Meier survival analysis diagram with overall survival as the primary endpoint after the gene combination 1 of the invention is used for grading the malignant degree of renal cancer in Experiment Example 2 of the invention;
Figure 7 is the Kaplan-Meier survival analysis diagram with tumor specific survival as the primary endpoint after the gene combination in Example 1 of the present invention is used for grading the malignant degree of pan-cancer in Experiment Example 3 of the present invention;
Figure 8 is the Kaplan-Meier survival analysis diagram with progression-free survival as the primary endpoint after the gene combination in Example 1 of the present invention is used for grading the malignant degree of pan-cancer in Experiment Example 3 of the present invention;
Figure 9 is the Kaplan-Meier survival analysis diagram with disease-free survival as the primary endpoint after the gene combination in Example 1 of the present invention is used for grading the malignant degree of pan-cancer in Experiment Example 3 of the present invention;
Figure 10 is the Kaplan-Meier survival analysis diagram with overall survival as the primary endpoint after the gene combination in Example 1 of the present invention is used for grading the malignant degree of pan-cancer in Experiment Example 3 of the present invention;
Figure 11 is the Kaplan-Meier survival analysis diagram with tumor specific survival as the primary endpoint after the gene combination 1 in Experiment Example 2 of the present invention is used for grading the malignant degree of pan-cancer in Experiment Example 4 of the present invention;
Figure 12 is the Kaplan-Meier survival analysis diagram with tumor progression-free survival as the primary endpoint after the gene combination 1 in Experiment Example 2 of the present invention is used for grading the malignant degree of pan-cancer in Experiment Example 4 of the present invention;
Figure 13 is the Kaplan-Meier survival analysis diagram with tumor overall survival as the primary endpoint after the gene combination 1 in Experiment Example 2 of the present invention is used for grading the malignant degree of pan-cancer in Experiment Example 4 of the present invention.

### DETAILED DESCRIPTION

The following embodiments are provided for a better understanding of the present invention, are not limited to the best implementations, and do not constitute a limitation on the content or scope of protection of the present invention. Any product identical or similar to the present invention, derived by anyone under the inspiration of the present invention or by combining the present invention with other features of the prior art, falls within the scope of protection of the present invention.

Where specific experimental steps or conditions are not indicated in the embodiments, the operations or conditions of conventional experimental steps described in the documents in the art can be followed. The reagents or instruments used without the manufacturer indicated are conventional reagent products that can be commercially available.

### Example 1 Gene combination (Panel) for human tumor grading

The inventor mainly used the high throughput database of renal cancer exome sequencing of Peking University First Hospital to screen, and confirmed a gene combination (panel) for human tumor grading, which comprises gene set A and gene fragment set B;

the gene set A comprises at least one selected from ASAH1, ASXL1, BCOR, BRAF, CALML6, CCDC136, CIDEC, COX18, CSF1R, CYP3A5, DEK, DNMT3A, EGR1, FAM71E2, FGFR1, FKBP7, FLT1, FLT3, FLT4, GLIS1, IDH2, IFITM3, IMMT, KDR, KIT, KMT2A, KNOP1, KRT76, KRT9, KRTAP10-10, KRTAP10-8, MAF, MECOM, MFRP, MLLT3, MNS1, MRTFA, MTOR, MYH11, NF1, NUP214, PDGFRA, PDGFRB, PML, PRB2, PROSER3, RAF1, RARA, RBM15, RET, REXO1, RPN1, RUNX1T1, SCYL1, SLC16A6, SRC, STAG2, TCEAL5, TET2, TMEM82, TP53, TRIM26, U2AF1, U2AF2, UGT1A1, USP35, VEGFA, WBP2NL, WDR44, ZNF20, ZNF700 and ZRSR2;
the gene fragment set B comprises at least one selected from chr2:179479501-179610249, chr2:207989501-208000249, chr2:219719501-219840249, chr2:3679501-3700249, chr3:126249501-126270249, chr3:129319501-129330249, chr3:138659501-138770249, chr3:183999501-184020249, chr4:1189501-1230249, chr4:8579501-8590249, chr4:9319501-9330249, chr5:150899501-150940249, chr6:147819501-147840249, chr6:157089501-157110249, chr6:164889501-164900249, chr6:20399501-20410249, chr6:26519501-26530249, chr6:71659501-71670249, chr6:73329501-73340249, chr7:100539501-100560249, chr8:1939501-1960249, chr8:21999501-22070249, chr8:29189501-29200249, chr9:91789501-91800249, chr10:99419501-99440249, chr11:17739501-17760249, chr11:63329501-63350249, chr12:169501-250249, chr12:54329501-54350249, chr12:63179501-63550249, chr12:7269501-7310249, chr13:114519501-114530249, chr15:73649501-73670249, chr15:74209501-74220249, chr15:78409501-78430249, chr15:83859501-83880249, chr18:8809501-8820249, chr19:24059501-24070249, chr19:4229501-4250249, chr19:46879501-46900249, chr20:22559501-22570249, chr20:62189501-62200249, chr21:45949501-46110249, chr22:19499501-19760249, chr22:36649501-38700249 and chr22:46309501-47080249; and the position of the gene fragment in the gene fragment set B is annotated with GRCh37 as the standard. In GRCh38 or a new version of the Genome Reference Consortium Human Build in the future, the number may change, but the location of the objective fragment pointed to and the genes available for detection will not change.

Optionally, the detailed genes included in the gene fragment set B are as follows:

**Table 1 Gene Fragment Set B**

| position of gene fragment | genes available for detection |
|---|---|
| chr2:179479501-179610249 | at least one of TTN, MIR548N and LOC100506866 |
| chr2:207989501-208000249 | KLF7 |
| chr2:219719501-219840249 | at least one of WNT6, CDK5R2 and WNT10A |
| chr2:3679501-3700249 | COLEC11 |
| chr3:126249501-126270249 | at least one of C3orf22 and CHST13 |
| chr3:129319501-129330249 | PLXND 1 |
| chr3:138659501-138770249 | at least one of FOXL2, PRR23B, PRR23C, C3orf72 and PRR23A |
| chr3:183999501-184020249 | at least one of PSMD2 and ECE2 |
| chr4:1189501-1230249 | at least one of CTBP1, SPON2 and LOC100130872 |
| chr4:8579501-8590249 | GPR78 |
| chr4:9319501-9330249 | at least one of LOC728369, LOC728373, LOC728379, USP17L5, LOC728393, LOC728400 and LOC728405 |
| chr5:150899501-150940249 | FAT2 |
| chr6:147819501-147840249 | SAMD5 |
| chr6:157089501-157110249 | at least one of ARID1B and MIR4466 |
| chr6:164889501-164900249 | C6orf118 |
| chr6:20399501-20410249 | E2F3 |
| chr6:26519501-26530249 | HCG11 |
| chr6:71659501-71670249 | B3GAT2 |
| chr6:73329501-73340249 | KCNQ5 |
| chr7:100539501-100560249 | ACHE |
| chr8:1939501-1960249 | KBTBD 11 |
| chr8:21999501-22070249 | at least one of BMP1, SFTPC and LGI3 |
| chr8:29189501-29200249 | DUSP4 |
| chr9:91789501-91800249 | SHC3 |
| chr 10:99419501-99440249 | at least one of PI4K2A and AVPI1 |
| chr11:17739501-17760249 | at least one of KCNC1 and MYOD1 |
| chr11:63329501-63350249 | at least one of PLA2G16 and PLAAT2 |
| chr12:169501-250249 | at least one of IQSEC3 and LOC574538 |
| chr12:54329501-54350249 | at least one of HOXC12 and HOXC13 |
| chr12:63179501-63550249 | at least one of AVPR1A and PPM1H |
| chr12:7269501-7310249 | at least one of CLSTN3, RBP5 and MATL2963 |
| chr13:114519501-114530249 | GAS6 |
| chr15:73649501-73670249 | HCN4 |
| chr15:74209501-74220249 | at least one of LOXL1 and LOC100287616 |
| chr15:78409501-78430249 | CIB2 |
| chr15:83859501-83880249 | HDGFL3 |
| chr18:8809501-8820249 | MTCL1 |
| chr 19:24059501-24070249 | ZNF726 |
| chr19:4229501-4250249 | at least one of EBI3, CCDC94 |
| chr19:46879501-46900249 | PPP5C |
| chr20:22559501-22570249 | FOXA2 |
| chr20:62189501-62200249 | HELZ2 |
| chr21:45949501-46110249 | at least one of TSPEAR, KRTAP12-2, KRTAP12-1, KRTAP10-10, KRTAP10-4, KRTAP10-6, KRTAP10-7, KRTAP10-9, KRTAP10-1, KRTAP10-11, KRTAP10-2, KRTAP10-5, KRTAP10-8, KRTAP10-3, KRTAP12-3 and KRTAP12-4 |
| chr22:19499501-19760249 | at least one of GP1BB, SEPTIN5, TBX1, CLDN5, CDC45, LOC150185 and SEPT5-GP1BB |
| chr22:36649501-38700249 | at least one of hsa-mir-659, CSF2RB, CSNK1E, H1F0, IL2RB, LGALS1, LGALS2, MFNG, MPST, MYH9, NCF4, POLR2F, PVALB, RAC2, SOX10, SSTR3, TST, PLA2G6, GALR3, APOL1, EIF3D, PICK1, CACNG2, IFT27, TRIOBP, CDC42EP1, GCAT, SLC16A8, SH3BP1, MAFF, TXN2, TMEM184B, GGA1, CYTH4, CARDIO, EIF3L, PDXP, NOL12, KCTD17, FOXRED2, BAIAP2L2, C22orf23, MICALL1, ELFN2, C1QTNF6, ANKRD54, TMPRSS6, C22orf33, MIR658, MIR659, LOC100506241 and MIR4534 |
| chr22:46309501-47080249 | at least one of hsa-let-7b, PPARA, WNT7B, CELSR1, PKDREJ, GRAMD4, GTSE1, TTC38, C22orf26, TRMU, LOC150381, C22orf40, CN5H6.4, MIRLET7BHG, MIRLET7A3, MIRLET7B, LOC730668, LOC100271722, MIR3619 and MIR4763 |

### Example 2 A method for grading the malignancy degree and predicting the prognosis of human renal cancer

This example provides a method for grading detection of human tumor, comprising using the gene combination (panel) in Example 1 to perform human renal cancer malignancy grading and prognosis predication, and the specific steps are as follows:
(1) Renal cancer tissue and healthy control tissue samples were taken, the renal cancer tissue samples can be renal cancer cell lines, fresh renal cancer samples, frozen renal cancer samples or paraffin embedded renal cancer samples; the healthy control tissue can be the tissue of a known and recognized healthy person, or the adjacent tissue of the patient with renal cancer. In this example, paraffin embedded renal cancer specimens were selected, and normal adjacent tissues were used for healthy control tissues. DNA was extracted by conventional methods, and a library was constructed by conventional methods. Finally, the gene combination (panel) in Example 1 was used for targeted high-throughput sequencing to compare the sequencing data of renal cell carcinoma tissues and healthy tissues. The gene mutation and copy number variation (CNV) of each gene in gene set A and the copy number variation (CNV) of each gene in gene fragment set B in gene combination of renal cancer tissue were obtained.
   The gene mutation comprises base replacement mutation, deletion mutation, insertion mutation and fusion mutation, and the gene copy number variation includes gene copy number increase and gene copy number decrease.
(2) Judgment based on the mutation and/or variation of each gene in the gene combination of renal cancer tissue obtained in step (1):
   If at least one gene in gene set A has gene mutation or copy number variation, or at least one fragment in gene fragment set B has copy number increase, the renal cancer patient is graded as a high-risk group with a worse tumor prognosis. Conversely, if there is no gene mutation or copy number variation in gene set A, and no gene copy number increase in any fragment in gene fragment set B, and the renal cancer patient is graded as a low-risk group with good tumor prognosis.

### Example 3

As an alternative embodiment of Example 2, in the present invention, the genes in the gene combination (panel) of Example 1 are allowed to be picked and recombined to form a new gene combination. The evaluation criterion is that, if at least one gene selected from gene set A has gene mutation or copy number variation, the renal cancer patient is graded as a high-risk group; for the gene fragment selected from gene fragment set B, if at least one of the gene fragments in the region increased copy number, indicating that the renal cancer patient is graded as a high-risk group. Conversely, there is no gene mutation or copy number variation in gene set A, and no gene copy number increase in any fragment selected from gene fragment set B, and the renal cancer patient is graded as the low-risk group.

### Example 4 A method for grading and predicting the prognosis of human pan-cancer

This example provides a method for grading and detecting human tumor, including, using the gene combination (panel) in Example 1 to grade and predict the prognosis of human pan-cancer, and the specific steps are as follows:
(1) Pan-cancer (the pan-cancer herein is defined as all types of cancer in TCGA pan-cancer data, comprising adrenal cancer, urothelial cancer, breast cancer, cervical cancer, cholangiocarcinoma, colon cancer, lymphoma, esophageal cancer, glioblastoma, head and neck squamous cell carcinoma, chromophobe cell renal carcinoma, renal clear cell carcinoma, renal papillary cell carcinoma, leukemia, glioma, hepatocellular carcinoma, lung adenocarcinoma, lung squamous cell carcinoma, mesothelioma, ovarian serous cystadenocarcinoma, pancreatic cancer, pheochromocytoma and paraganglioma, prostate cancer, rectal cancer, sarcoma, cutaneous melanoma, gastric cancer, testicular cancer, thyroid cancer, thymic cancer, endometrial cancer, uterine sarcoma, uveal melanoma. The pan-cancer mentioned in this example is defined as such and will not be repeated) tissue and healthy control tissue specimens were taken. The pan-cancer tissue specimens can be pan-cancer cell lines, fresh pan-cancer specimens, frozen pan-cancer specimens or parafin-embedded pan-cancer specimens; healthy control tissues can be tissues from known healthy people, or paracancerous tissues from pan-cancer patients themselves. In this example, paraffin-embedded pan-cancer specimens were selected, and normal paracancerous tissues were used as healthy control tissues. DNA was extracted by conventional methods, and a library was constructed by conventional methods. Finally, the gene combination (panel) in Example 1 was used for targeted high-throughput sequencing to compare the sequencing data of pan-cancer tissues and healthy tissues. The gene mutation and copy number variation (CNV) of each gene in gene set A and the copy number variation (CNV) of each gene in gene fragment set B in gene combination of renal cancer tissue were obtained.
   The gene mutation comprises base replacement mutation, deletion mutation, insertion mutation and fusion mutation, and the gene copy number variation includes gene copy number increase and gene copy number decrease.
(2) Judgment based on the mutation and/or variation of each gene in the gene combination of pan-cancer tissues obtained in step (1):
   If at least one gene in gene set A has gene mutation or copy number variation, or at least one fragment in gene fragment set B has copy number increase, the pan-cancer patient is graded as a high-risk group with a worse tumor prognosis. Conversely, if there is no gene mutation or copy number variation in gene set A, and no gene copy number increase in any fragment in gene fragment set B, and the pan-cancer patient is graded as a low-risk group with good tumor prognosis.

### Example 5

As an alternative embodiment of Example 4, in the present invention, the genes in the gene combination (panel) of Example 1 are allowed to be picked and recombined to form a new gene combination. The evaluation criterion is that, if at least one gene selected from gene set A has gene mutation or copy number variation, the pan-cancer patient is graded as a high-risk group; for the gene fragment selected from gene fragment set B, if at least one of the gene fragments in the region increased copy number, indicating that, the pan-cancer patient is graded as a high-risk group. Conversely, there is no gene mutation or copy number variation in gene set A, and no gene copy number increase in any fragment selected from gene fragment set B, and the pan-cancer patient is graded as a low-risk group.

### Experiment Example 1 The feasibility verification of gene combination and detection method for human tumor grading in evaluating the malignant grade and prognosis prediction of human renal clear cell carcinoma

Clear cell carcinoma is the most common pathological type of renal cancer, accounting for 70% or more of all renal cancers. TCGA (Pan-cancer Atlas) renal clear cell carcinoma database is a globally recognized renal cancer database, which can be used for testing the feasibility and reliability of the invention for evaluating the malignant degree grading and prognosis prediction of renal cancer.

TCGA (Pan-cancer Atlas) renal clear cell carcinoma data has 512 patient data, of which 354 patients have complete gene mutation and copy number variation data, which is applicable to the application conditions of the present invention.

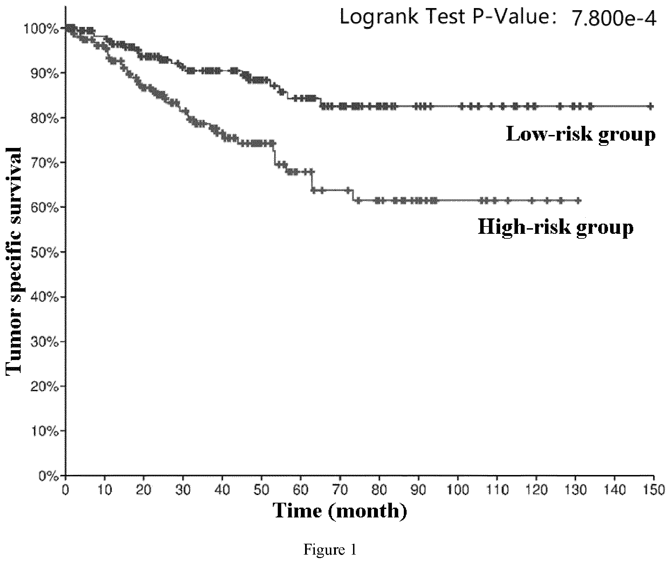

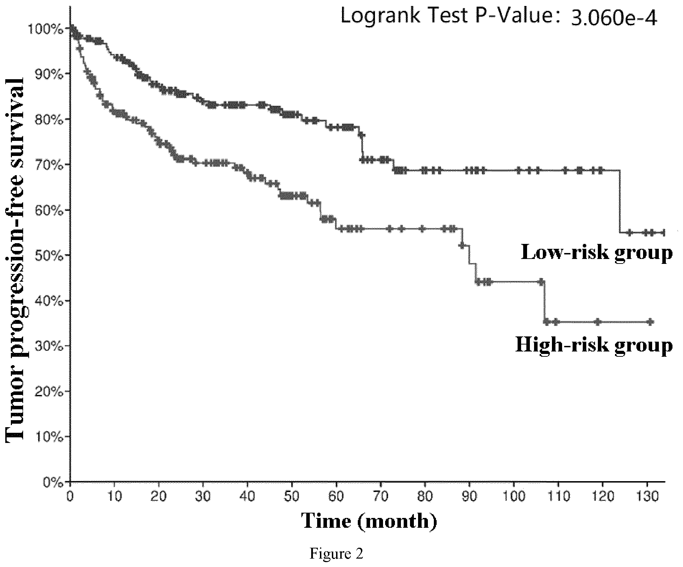

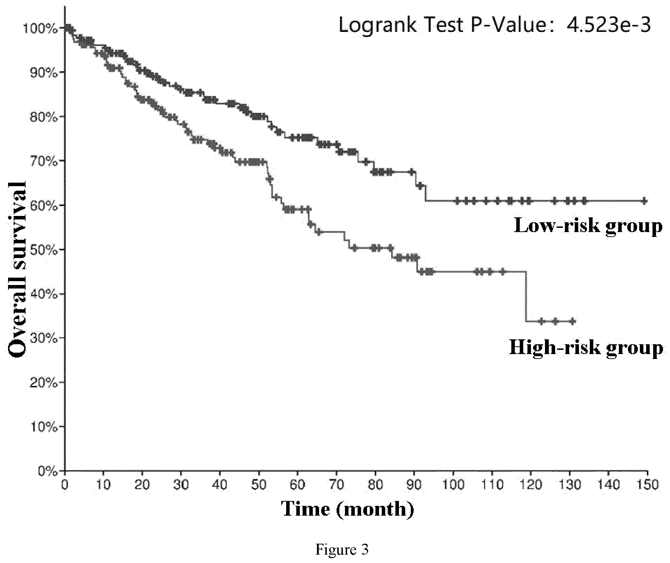

According to the method described in Example 2, all genes of gene set A and all fragments of gene fragment set B in Example 1 are selected for implementation in this experimental example. The actual genes used for detection in gene fragment set B are shown in Table 2. The above 354 patients were classified into high-risk group and low-risk group. The high-risk group accounted for 46.6% and the low-risk group accounted for 53.4%. Through Kaplan-Meier survival analysis, it can be seen that the tumor specific survival (Figure 1), tumor progression-free survival (Figure 2) and total survival (Figure 3) of the high-risk group and the low-risk group have statistical differences and meet the grouping expectations of the invention: the low-risk group has significantly better tumor specific survival (Log-rank p value=7.800e-4), tumor progression-free survival (Log-rank p value=3.060e-4) and total survival (Log-rank p value=4.523e-3). Therefore, it is accurate and reliable to use the gene combination of the invention to grade the malignancy and predict the prognosis of renal cancer patients.

**Table 2 Genes actually tested against Gene Fragment Set B in Experiment Example 1**

| Position of gene fragment | Genes available for detection in Experiment Example |
|---|---|
| chr2:179479501-179610249 | TTN |
| chr2:207989501-208000249 | KLF7 |
| chr2:219719501-219840249 | WNT6 |
| chr2:3679501-3700249 | COLEC11 |
| chr3:126249501-126270249 | CHST13 |
| chr3:129319501-129330249 | PLXND 1 |
| chr3:138659501-138770249 | FOXL2 |
| chr3:183999501-184020249 | PSMD2 |
| chr4:1189501-1230249 | CTBP1 |
| chr4:8579501-8590249 | GPR78 |
| chr4:9319501-9330249 | USP17L5 |
| chr5:150899501-150940249 | FAT2 |
| chr6:147819501-147840249 | SAMD5 |
| chr6:157089501-157110249 | ARID1B |
| chr6:164889501-164900249 | C6orf118 |
| chr6:20399501-20410249 | E2F3 |
| chr6:26519501-26530249 | HCG11 |
| chr6:71659501-71670249 | B3GAT2 |
| chr6:73329501-73340249 | KCNQ5 |
| chr7:100539501-100560249 | ACHE |
| chr8:1939501-1960249 | KBTBD11 |
| chr8:21999501-22070249 | BMP1 |
| chr8:29189501-29200249 | DUSP4 |
| chr9:91789501-91800249 | SHC3 |
| chr 10:99419501-99440249 | PI4K2A |
| chr11:17739501-17760249 | MYOD 1 |
| chr11:63329501-63350249 | PLAAT2 |
| chr12:169501-250249 | IQSEC3 |
| chr12:54329501-54350249 | HOXC13 |
| chr12:63179501-63550249 | AVPR1A |
| chr12:7269501-7310249 | CLSTN3 |
| chr13:114519501-114530249 | GAS6 |
| chr15:73649501-73670249 | HCN4 |
| chr15:74209501-74220249 | LOXL1 |
| chr15:78409501-78430249 | CIB2 |
| chr15:83859501-83880249 | HDGFL3 |
| chr18:8809501-8820249 | MTCL1 |
| chr 19:24059501-24070249 | ZNF726 |
| chr19:4229501-4250249 | EBI3 |
| chr19:46879501-46900249 | PPP5C |
| chr20:22559501-22570249 | FOXA2 |
| chr20:62189501-62200249 | HELZ2 |
| chr21:45 9495 01-46110249 | TSPEAR |
| chr22:19499501-19760249 | SEPTIN5 |
| chr22:36649501-38700249 | MYH9 |
| chr22:46309501-47080249 | WNT7B |

### Experimental Example 2 The feasibility verification of the preferred gene combination and detection method in evaluating the malignant grade and prognosis prediction of human renal clear cell carcinoma

The present invention allows to select any gene fragments from the gene combination (panel) and combine to form a new gene combination, and use the same judgment criteria to grade the malignancy of renal cell carcinoma and predict the prognosis of the tumor. Here, gene set A1 is selected from gene set A of gene combination (panel), and gene fragment set B 1 is selected from gene fragment set B to form gene combination 1 (panel 1), which is used for malignant grade and prognosis prediction of renal cell carcinoma, and feasibility analysis is performed using TCGA (Pan-cancer Atlas) renal clear cell carcinoma database. Similarly, the judgment criteria are: if there is a gene mutation or copy number variation of at least one gene in gene set A1, or there is an increase in the copy number of at least one region in gene fragment set B 1, it indicates that the renal cancer patients are graded as a high-risk group with a worse tumor prognosis; conversely, if there is no gene mutation or copy number variation in gene set A1, and there is no gene copy number increase in gene fragment set B1, the patients with this type of renal cell carcinoma are graded as a the low-risk group with a good tumor prognosis. It should be specially noted that in this experimental example, gene combination 1 (panel 1) is optimized on the basis of gene combination (panel), which has higher accuracy (higher specificity). Compared with gene combination 1 (panel 1), gene combination (panel) has a wider range of application (higher sensitivity).

**Table 3 Gene Set A1**

| Gene name |
|---|
| ASAH1, CCDC136, FAM71E2, IFITM3, KRT9, PRB2, PROSER3, TCEAL5, U2AF2, USP35, WDR44 and ZNF700 |

**Table 4 Gene Fragment Set B 1**

| Position of gene fragment | Genes available for detection in Experiment Example |
|---|---|
| chr2:179479501-179610249 | TTN |
| chr2:207989501-208000249 | KLF7 |
| chr2:219719501-219840249 | WNT6 |
| chr3:126249501-126270249 | CHST13 |
| chr3:129319501-129330249 | PLXND 1 |
| chr3:138659501-138770249 | FOXL2 |
| chr3:183999501-184020249 | PSMD2 |
| chr5:150899501-150940249 | FAT2 |
| chr7:100539501-100560249 | ACHE |
| chr13:114519501-114530249 | GAS6 |

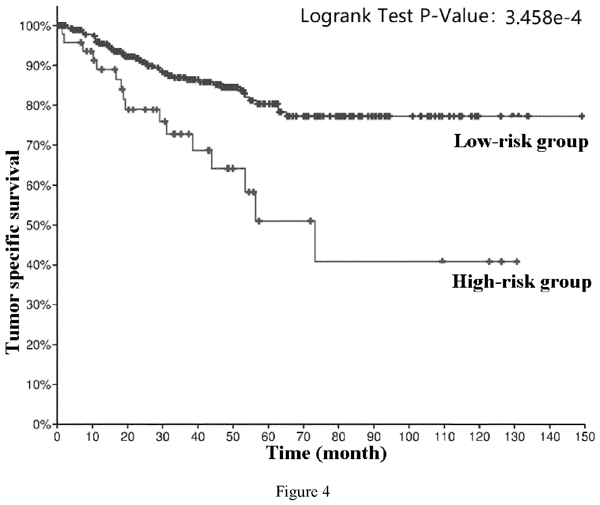

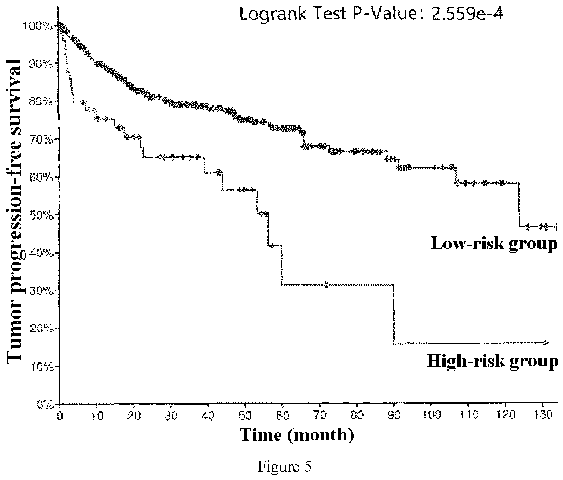

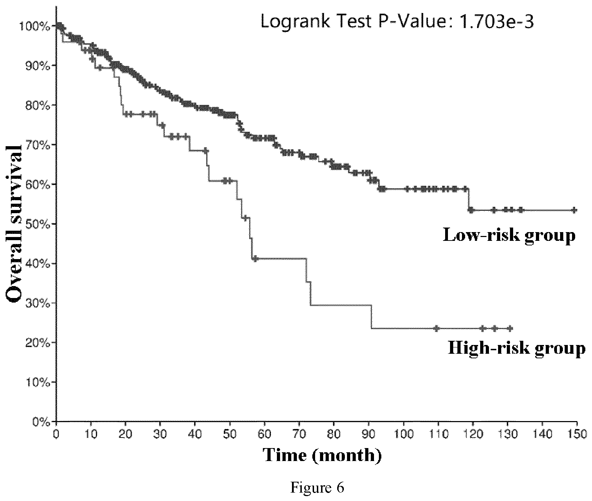

According to the method described in Example 2, the above-mentioned 354 patients were graded by the gene combination 1 (panel 1) into malignant degree, and successfully divided into high-risk group and low-risk group, of which the high-risk group accounted for 14.4% and the low-risk group accounted for 85.6%. Through Kaplan-Meier survival analysis, it can be seen that there are statistical differences in the tumor-specific survival of the high-risk group and low-risk group (Figure 4), tumor progression-free survival (Figure 5) and overall survival (Figure 6) between high-risk group and low-risk group, and it meets the grouping expectations of the invention: the low-risk group has significantly better tumor-specific survival (Log-rank p value=3.458e-4), tumor progression-free survival (Log-rank p value=2.559e-4) and total survival (Log-rank p value=1.703e-3). Therefore, other gene combinations selected from the gene combination (panel) by present invention can still be used for grading the malignancy and predicting the renal cancer patient prognosis.

### Experimental Example 3 The feasibility verification of gene combination and detection methods used for human tumor grading in evaluating the malignant grade and prognosis prediction of human pan-cancer

TCGA (Pan-cancer Atlas) pan-cancer database is a globally recognized pan-cancer database, which can be used to test the feasibility and credibility of the invention for evaluating the grade of malignancy and prognosis prediction of pan-cancer.

TCGA (Pan-cancer Atlas) pan-cancer data has 10967 pan-cancer data, of which 9896 have complete gene mutation and copy number variation data, which is applicable to the application conditions of the invention.

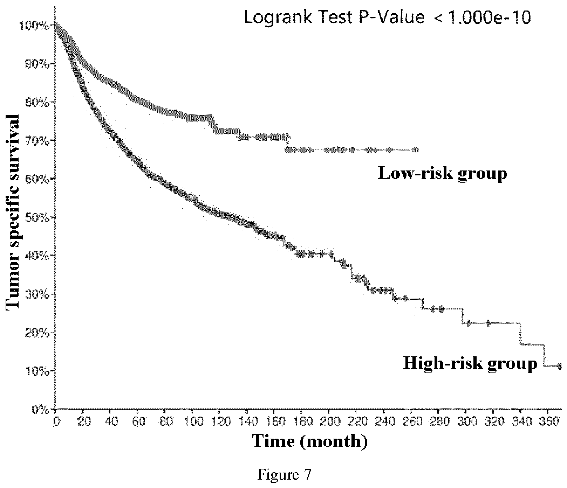

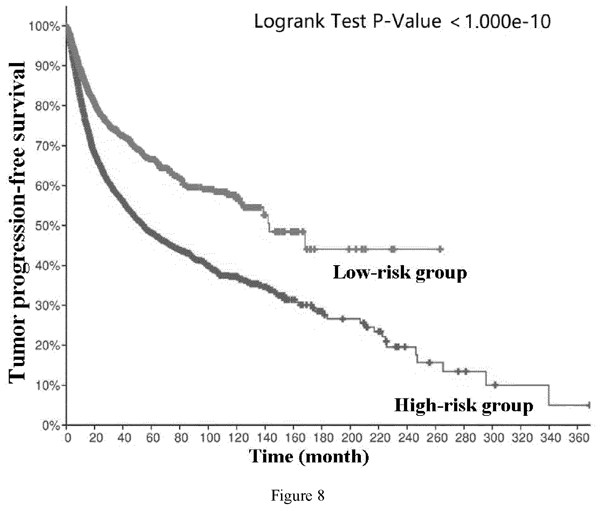

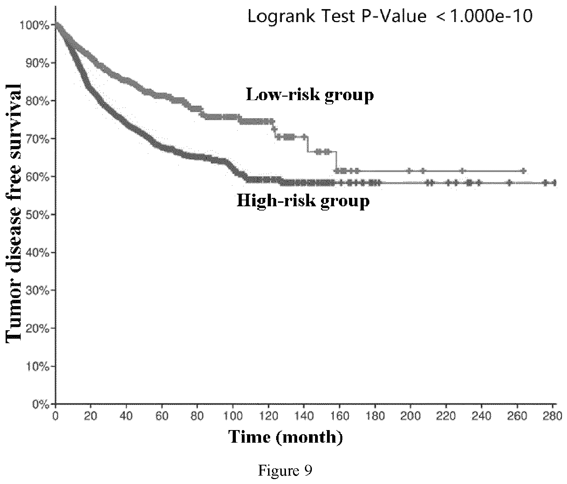

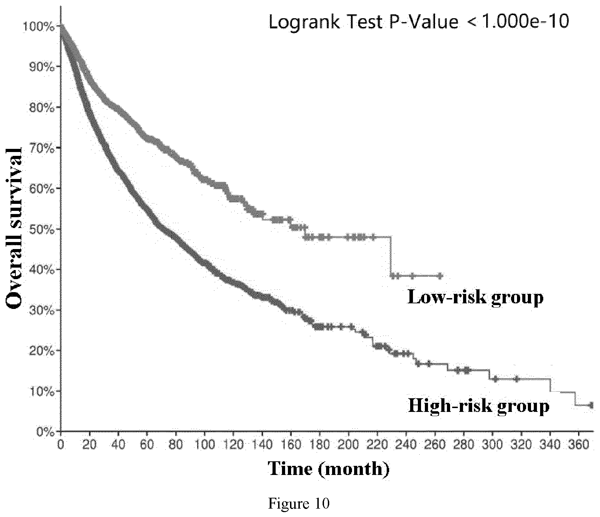

According to the method described in Example 4, all genes in gene set A and all fragments in gene fragment set B in Example 1 were selected for implementation in this experiment. The genes actually used for detection in gene fragment set B are the same as those in Table 2 in Example 1. The above 9896 patients were divided into high-risk group and low-risk group according to the grade of malignancy. The high-risk group accounted for 77.0%, and the low-risk group accounted for 23.0%. Through Kaplan-Meier survival analysis, we can see the tumor-specific survival (Figure 7), tumor progression-free survival (Figure 8) Tumor disease-free survival (Figure 9) and total survival (Figure 10) have statistical differences and meet the grouping expectations of the invention: the low-risk group has significantly better tumor-specific survival (Log-rank p value<1.000e-10), tumor progression-free survival (Log-rank p value<1.000e-10), tumor disease-free survival (Log-rank p value<1.000e-10) and total survival (Log-rank p value<1.000e-10). Therefore, it is accurate and reliable to use the gene combination of the invention for grading the malignancy and predicting the prognosis of pan-cancer patients.

### Experimental Example 4 The feasibility verification of the preferred gene combination and detection method in evaluating the malignant grade and prognosis prediction of human pan-cancer

The present invention allows to select any gene fragments from the gene combination (panel) for combination to form a new gene combination, and use the same judgment criteria for grading the pan-cancer malignancy and predicting tumor prognosis. Here, according to the method described in Example 5, the gene combination 1 (panel 1) in Experiment Example 2 is selected and used grading the pan-cancer malignancy and predicting pan-cancer prognosis, and the feasibility analysis is performed using TCGA (Pan-cancer Atlas) pan-cancer database. Similarly, the judgment criteria are: If at least one gene in gene set A1 has gene mutation or copy number variation, or at least one fragment in gene fragment set B1 has copy number increase, it indicates that the pan-cancer patients are at high-risk and have a worse tumor prognosis; conversely, if there is no gene mutation or copy number variation in gene set A1, and no gene copy number increase in any fragment in gene fragment set B1, then this type of pan-carcinoma patient is a low-risk group with good tumor prognosis. It should be specially noted that in this experimental example, gene combination 1(panel 1) is optimized on the basis of gene combination (panel), which has significantly lower implementation cost due to fewer detection sites.

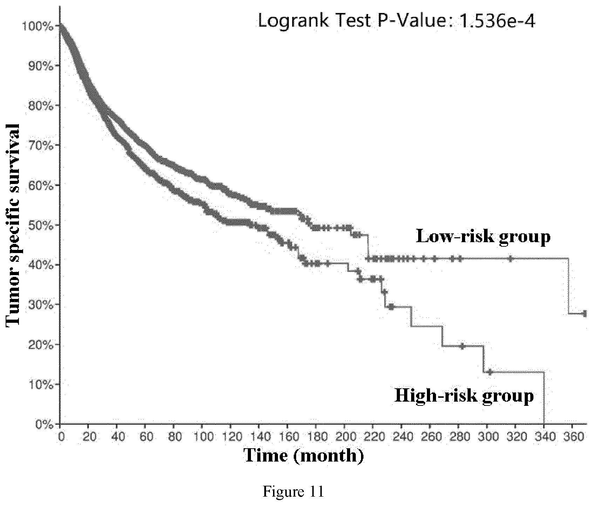

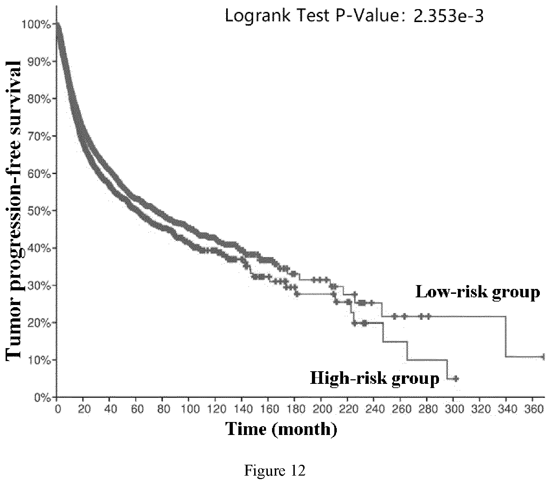

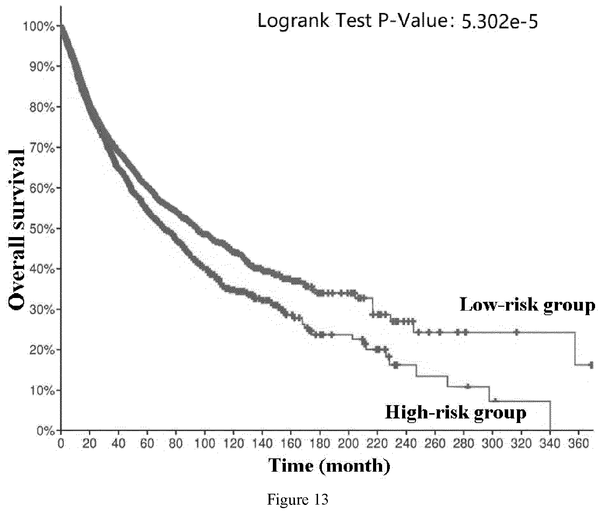

According to the method described in Example 4, gene combination 1 (panel 1) graded the malignant degree of 9896 patients in the above-mentioned pan-cancer database, and successfully divided them into high-risk group and low-risk group, of which the high-risk group accounted for 26.8% and the low-risk group accounted for 73.2%. Through Kaplan-Meier survival analysis, it can be seen that there are statistical differences in the tumor-specific survival of the high-risk group and the low-risk group (Figure 11), the tumor progression-free survival (Figure 12) and total survival (Figure 13) between high-risk group and low-risk group,, and it meets the grouping expectations of the present invention: the low-risk group has significantly better tumor-specific survival (Log-rank p value=1.536e-4), tumor progression-free survival (Log-rank p value=2.353e-3) and total survival (Log-rank p value=5.302e-5). Therefore, other gene combinations selected from the gene combination (panel) by the present invention can still be used for grading the malignancy and predicting the prognosis of pan-cancer patients.

Obviously, the above-mentioned embodiments are merely examples made for clear description, but do not limit implementations. For those skilled in the art, other different forms of variations or modifications can also be made on the basis of the above-mentioned description. All embodiments are not necessary to be and cannot be exhaustively listed herein. In addition, the obvious variations or modifications derived therefrom all fall within the scope of protection of the present invention.

## Claims

1. A gene combination for human tumor grading, wherein the gene combination consists of a gene set A and a gene fragment set B;
the gene set A comprises at least one selected from ASAH1, ASXL1, BCOR, BRAF, CALML6, CCDC136, CIDEC, COX18, CSF1R, CYP3A5, DEK, DNMT3A, EGR1, FAM71E2, FGFR1, FKBP7, FLT1, FLT3, FLT4, GLIS1, IDH2, IFITM3, IMMT, KDR, KIT, KMT2A, KNOP1, KRT76, KRT9, KRTAP10-10, KRTAP10-8, MAF, MECOM, MFRP, MLLT3, MNS1, MRTFA, MTOR, MYH11, NF1, NUP214, PDGFRA, PDGFRB, PML, PRB2, PROSER3, RAF1, RARA, RBM15, RET, REXO1, RPN1, RUNX1T1, SCYL1, SLC16A6, SRC, STAG2, TCEAL5, TET2, TMEM82, TP53, TRIM26, U2AF1, U2AF2, UGT1A1, USP35, VEGFA, WBP2NL, WDR44, ZNF20, ZNF700 and ZRSR2;
the gene fragment set B comprises at least one selected from chr2:179479501-179610249, chr2:207989501-208000249, chr2:219719501-219840249, chr2:3679501-3700249, chr3:126249501-126270249, chr3:129319501-129330249, chr3:138659501-138770249, chr3:183999501-184020249, chr4:1189501-1230249, chr4:8579501-8590249, chr4:9319501-9330249, chr5:150899501-150940249, chr6:147819501-147840249, chr6:157089501-157110249, chr6:164889501-164900249, chr6:20399501-20410249, chr6:26519501-26530249, chr6:71659501-71670249, chr6:73329501-73340249, chr7:100539501-100560249, chr8:1939501-1960249, chr8:21999501-22070249, chr8:29189501-29200249, chr9:91789501-91800249, chr10:99419501-99440249, chr11:17739501-17760249, chr11:63329501-63350249, chr12:169501-250249, chr12:54329501-54350249, chr12:63179501-63550249, chr12:7269501-7310249, chr13:114519501-114530249, chr15:73649501-73670249, chr15:74209501-74220249, chr15:78409501-78430249, chr15:83859501-83880249, chr18:8809501-8820249, chr19:24059501-24070249, chr19:4229501-4250249, chr19:46879501-46900249, chr20:22559501-22570249, chr20:62189501-62200249, chr21:45949501-46110249, chr22:19499501-19760249, chr22:36649501-38700249 and chr22:46309501-47080249; and the position of the gene fragment in the gene fragment set B is annotated with GRCh37 as the standard.

2. A gene combination for human tumor grading of claim 1, wherein the detailed genes included in the gene fragment set B are as follows:
**Table 1 Gene Fragment Set B**
| position of gene fragment | genes available for detection |
|---|---|
| chr2:179479501-179610249 | at least one of TTN, MIR548N and LOC100506866 |
| chr2:207989501-208000249 | KLF7 |
| chr2:219719501-219840249 | at least one of WNT6, CDK5R2 and WNT10A |
| chr2:3679501-3700249 | COLEC11 |
| chr3:126249501-126270249 | at least one of C3orf22 and CHST13 |
| chr3:129319501-129330249 | PLXND 1 |
| chr3:138659501-138770249 | at least one of FOXL2, PRR23B, PRR23C, C3orf72 and PRR23A |
| chr3:183999501-184020249 | at least one of PSMD2 and ECE2 |
| chr4:1189501-1230249 | at least one of CTBP1, SPON2 and LOC100130872 |
| chr4:8579501-8590249 | GPR78 |
| chr4:9319501-9330249 | at least one of LOC728369, LOC728373, LOC728379, USP17L5, LOC728393, LOC728400 and LOC728405 |
| chr5:150899501-150940249 | FAT2 |
| chr6:147819501-147840249 | SAMD5 |
| chr6:157089501-157110249 | at least one of ARID1B and MIR4466 |
| chr6:164889501-164900249 | C6orf118 |
| chr6:20399501-20410249 | E2F3 |
| chr6:26519501-26530249 | HCG11 |
| chr6:71659501-71670249 | B3GAT2 |
| chr6:73329501-73340249 | KCNQ5 |
| chr7:100539501-100560249 | ACHE |
| chr8:1939501-1960249 | KBTBD 11 |
| chr8:21999501-22070249 | at least one of BMP1, SFTPC and LGI3 |
| chr8:29189501-29200249 | DUSP4 |
| chr9:91789501-91800249 | SHC3 |
| chr 10:99419501-99440249 | at least one of PI4K2A and AVPI1 |
| chr11:17739501-17760249 | at least one of KCNC1 and MYOD1 |
| chr11:63329501-63350249 | at least one of PLA2G16 and PLAAT2 |
| chr12:169501-250249 | at least one of IQSEC3 and LOC574538 |
| chr12:54329501-54350249 | at least one of HOXC12 and HOXC13 |
| chr12:63179501-63550249 | at least one of AVPR1A and PPM1H |
| chr12:7269501-7310249 | at least one of CLSTN3, RBP5 and MATL2963 |
| chr13:114519501-114530249 | GAS6 |
| chr15:73649501-73670249 | HCN4 |
| chr15:74209501-74220249 | at least one of LOXL1 and LOC100287616 |
| chr15:78409501-78430249 | CIB2 |
| chr15:83859501-83880249 | HDGFL3 |
| chr18:8809501-8820249 | MTCL1 |
| chr 19:24059501-24070249 | ZNF726 |
| chr19:4229501-4250249 | at least one of EBI3, CCDC94 |
| chr19:46879501-46900249 | PPP5C |
| chr20:22559501-22570249 | FOXA2 |
| chr20:62189501-62200249 | HELZ2 |
| chr21:45949501-46110249 | at least one of TSPEAR, KRTAP12-2, KRTAP12-1, KRTAP10-10, KRTAP10-4, KRTAP10-6, KRTAP10-7, KRTAP10-9, KRTAP10-1, KRTAP10-11, KRTAP10-2, KRTAP10-5, KRTAP10-8, KRTAP10-3, KRTAP12-3 and KRTAP12-4 |
| chr22:19499501-19760249 | at least one of GP1BB, SEPTIN5, TBX1, CLDN5, CDC45, LOC150185 and SEPT5-GP1BB |
| chr22:36649501-38700249 | at least one of hsa-mir-659, CSF2RB, CSNK1E, H1F0, IL2RB, LGALS1, LGALS2, MFNG, MPST, MYH9, NCF4, POLR2F, PVALB, RAC2, SOX10, SSTR3, TST, PLA2G6, GALR3, APOL1, EIF3D, PICK1, CACNG2, IFT27, TRIOBP, CDC42EP1, GCAT, SLC16A8, SH3BP1, MAFF, TXN2, TMEM184B, GGA1, CYTH4, CARDIO, EIF3L, PDXP, NOL12, KCTD17, FOXRED2, BAIAP2L2, C22orf23, MICALL1, ELFN2, C1QTNF6, ANKRD54, TMPRSS6, C22orf33, MIR658, MIR659, LOC100506241 and MIR4534 |
| chr22:46309501-47080249 | at least one of hsa-let-7b, PPARA, WNT7B, CELSR1, PKDREJ, GRAMD4, GTSE1, TTC38, C22orf26, TRMU, LOC150381, C22orf40, CN5H6.4, MIRLET7BHG, MIRLET7A3, MIRLET7B, LOC730668, LOC100271722, MIR3619 and MIR4763 |
optionally, the gene set A comprises at least one selected from ASAH1, CCDC136, FAM71E2, IFITM3, KRT9, PRB2, PROSER3, TCEAL5, U2AF2, USP35, WDR44 and ZNF700; and
the gene fragment set B comprises at least one selected from chr2:179479501-179610249, chr2:207989501-208000249, chr2:21971-19840249, chr3:126249501-126270249, chr3:129319501-129330249, chr3:138659501-138770249, chr3:183999501-184020249, chr5:150899501-150940249, chr7: 100539501-100560249 and chr13: 114519501-114530249.

3. Use of the gene combination of claim 1 or 2 in the manufacture of products for human tumor grade detection.

4. The use of claim 3, wherein the tumor is a tumor of the urinary system or pan-cancer;
optionally, the tumor of the urinary system is malignant tumor of the urinary system;
optionally, the tumor of the urinary system is renal cancer; and
optionally, the pan-cancer is a cancer type in TCGA pan-cancer data.

5. The use of claim 3 or 4, wherein the tumor grading refers to the judgment of tumor malignancy and the prediction of tumor prognosis, which is used to guide clinical diagnosis and treatment; and
optionally, the tumor grading is divided into a high-risk group and a low-risk group.

6. The use of any one of claims 3-5, wherein the product comprises primers, probes, reagents, kits, gene chips or detection systems for detecting the genotypes of the genes in the gene combination.

7. The use of claim 6, wherein the product is used for detecting exon and related intron regions of genes in gene set A and gene fragment set B.

8. The use of any one of claims 5-7, wherein the method for grading the tumor comprises the following steps:
Step S1: evaluating the gene mutation and gene copy number variation of genes contained in the gene set A in the cancer cell tissue, and evaluating the gene copy number variation of the gene fragment set B in the cancer cell tissue; and
Step S2: judging the degree of malignancy of the cancer and predicting the prognosis of the tumor, based on the evaluation results of step S1.

9. The use of claim 8, wherein the gene mutation comprises base substitution mutation, deletion mutation, insertion mutation and/or fusion mutation, and the gene copy number variation comprises gene copy number increase and/or gene copy number decrease.

10. The use of claim 8 or 9, wherein, in the step S1, by comparing the sequencing data of the tumor tissue and the normal tissue, it is used for evaluating the gene mutation and copy number variation of the genes contained in the gene set A, while evaluating the copy number variation of the gene fragment set B.

11. The use of any one of claim 8-10, wherein in step S2, if at least one gene in gene set A has gene mutation or copy number variation, or at least one fragment in gene fragment set B has copy number increase, the tumor is graded as the high-risk group; on the contrary, there is no gene mutation or copy number variation in gene set A, and no gene copy number increase in any fragment in gene fragment set B, and the tumor is graded as the low-risk group.

12. The use of any one of claims 1-11, wherein any gene fragments are selected from the gene combination for combination to form a new gene combination, and the same tumor grading method is used for grading tumor malignancy and predicting tumor prognosis, so as to guide clinical diagnosis and treatment.
